# EUROPÄISCHE PATENTSCHRIFT

(11) **EP 2 252 264 B1**
(45) Veröffentlichungstag und Bekanntmachung des Hinweises auf die Patenterteilung: **01.08.2012**
(21) Anmeldenummer: 09706363.0
(22) Anmeldetag: 28.01.2009
(51) Int. Cl.: A61K 9/08

(54) **INJIZIERBARE BIOKOMPATIBLE ZUSAMMENSETZUNG**
INJECTABLE BIOCOMPATIBLE COMPOSITION
COMPOSITION BIOCOMPATIBLE INJECTABLE

(30) Priorität: 28.01.2008 DE 102008008071
(43) Veröffentlichungstag der Anmeldung: 24.11.2010
(73) Patentinhaber: NMI NATURWISSENSCHAFTLICHES UND MEDIZINISCHES INSTITUT AN DER UNIVERSITÄT TÜBINGEN, 72770 Reutlingen (DE)
(72) Erfinder: MOLLENHAUER, Jürgen, 72764 Reutlingen (DE); BENZ, Karin, 73037 Goeppingen (DE); PLATZ, Barbara, 55270 Zornheim (DE); WURST, Helmut, 72793 Pfullingen (DE); STOOP, Reinout, Stoop, NL-2251 PJ Voorschoten (NL)
(74) Vertreter: Witte, Weller & Partner
(86) Internationale Anmeldenummer: PCT/EP2009/000542
(87) Internationale Veröffentlichungsnummer: WO 2009/095223

(56) Entgegenhaltungen:
- WO-A-01/60335
- WO-A-01/66017
- WO-A-2009/010232
- US-A1- 2002 032 463
- US-A1- 2003 040 760
- ANSETH K S ET AL: "In situ forming degradable networks and their application in tissue engineering and drug delivery", JOURNAL OF CONTROLLED RELEASE, ELSEVIER, AMSTERDAM, NL, vol. 78, no. 1-3, 17 January 2002 (2002-01-17), pages 199-209, XP004329818, ISSN: 0168-3659, DOI: DOI:10.1016/S0168-3659(01)00500-4

## Beschreibung

Die vorliegende Erfindung betrifft eine injizierbare biokompatible Zusammensetzung, basierend auf einem polymeren Träger, der zumindest ein hydrophiles Polymer aufweist, wobei das Polymer *in situ* polymerisierbar ist, sowie die Verwendung dieser Zusammensetzung in der Medizin, und ein Verfahren zu dessen Herstellung.

Derartige injizierbare biokompatible Zusammensetzungen werden u.a. im Bereich der Medizin bzw. Chirurgie zu dem Zweck eingesetzt, degeneriertes oder verletztes Gewebe, bspw. Knorpelgewebe, eines Patienten zu ersetzen bzw. zu unterstützen. Insbesondere im Gebiet der Wiederherstellung von Gelenkknorpel werden gegenwärtig solche Zusammensetzungen eingesetzt, mit welchen Substanzen wie pharmakologische Wirkstoffe, Hormone, Enzyme, etc., aber auch Zellen, direkt in situ an der zu behandelnden Stelle bereit gestellt werden können, so dass die Bioverfügbarkeit solcher Substanzen oder der Zellen überhaupt erst ermöglicht bzw. erhöht wird. Solche Zusammensetzungen werden gegenwärtig v.a. im Gebiet der Knorpelregeneration eingesetzt.

Um Zellen in biokompatiblen, Gewebe-bildenden Matrizes, wie bspw. Hydrogele, einbetten zu können, sind im wesentlichen zwei Bedingungen zu beachten; die Gelierung darf durch die Anwesenheit von Zellen nicht unterbunden werden und die Vitalität der Zellen darf durch die Gelierung nicht beeinträchtigt werden. Diese Voraussetzungen müssen sowohl bei der Auswahl der verwendeten Materialien, als auch bei der Vernetzungsreaktion berücksichtigt werden.

Bisher sind Zellen in eine Reihe synthetischer Polymere, wie bspw. Polyethylenglykol, Polyvinylalkohol, etc., oder natürlicher Polymere, wie bspw. Agarose, Alginat, Chitosan, Kollagen, Hyaluronsäure, Fibrin, etc., eingebettet worden. Die Gelbildung bei synthetischen Polymeren kann durch eine chemische Reaktion erfolgen, bzw. auch durch Selbstassoziation, oder - wie es bei bestimmten Peptidsequenzen der Fall ist - durch Zugabe von Salz, oder durch eine Temperaturerhöhung. Auch natürlich vorkommende Polymere bilden oft Gele durch selbstassoziierende Reaktionen. Die Gelierung wird etwa bei Kollagen durch eine Erhöhung des pH-Werts, bei Agarose durch Temperaturerniedrigung, bei Alginat durch Kalziumzugabe induziert.

Ferner ist bspw. aus der WO 00/37124 bekannt, bestimmte Hyaluronsäure-Derivate als Gel bereit zu stellen, in das Chondrozyten dispergiert sind. Dieses Gel wird. Patienten zur Behandlung von u.a. chondralen und osteochondralen Schäden injiziert, wobei hier ausgenutzt wird, dass Hyaluronsäure als solche üblicherweise in allen Säugerarten vorkommt, und daher im Allgemeinen gut verträglich ist.

Die US 2002/0032463 A1 offenbart, ebenso wie die WO 01/66017 A1, eine biokompatible und abbaubare Hydrogel-Verbindung, die auf einem vernetzbaren Polymer aus Polyethylglykol basiert; in dieses Gel können dabei noch weitere Materialien eingebracht werden, wie bspw. Proteine, Antikörper, Hyaluronsäure, Chitosan, etc. Diese Verbindung soll dazu eingesetzt werden, um Blut- oder Fluidfluss aus Wunden zu stoppen, nachdem sie auf diese aufgebracht oder aufgesprüht wurde.

Die US 2003/0040760 A1 beschreibt ferner eine Zusammensetzung zum Wundverschluss, wobei hier eine Mischung aus einem hydrophilen, vernetzbaren Polymer eingesetzt wird, das ausgewählt ist aus bspw. Polyethylenglykol, Polyethylenoxid, PVA, etc.

Ferner sind Gele bekannt, bei welchen Chitosan bzw. Chitosan-Derivate als Basis-Trägermaterialien eingesetzt werden. So ist bspw. in der WO 99/997416 ein Gel offenbart, das auf einem Anteil Chitosan und einem Anteil eines Polyols basiert, und in welches Zellen eingekapselt werden können. Hier wird ausgenützt, dass die Zusammensetzung *in situ* geliert, und zwar ausgelöst durch bestimmte Temperaturen.

Abgesehen von der Injizierung bzw. Implantation von Gelen liegt gegenwärtig ein anderer Ansatz zur Behandlung von Knorpelschäden darin, dass Knorpelzellen entweder als Suspension freier Zellen in einen mit einer Membran bedeckten Knorpeldefekt im Gelenk eingespritzt werden oder indem die Zellen zunächst in einen Träger in vitro eingebracht werden und dann zusammen mit den Träger in den Defekt implantiert werden. Bei Bandscheibendefekten wurden die Zellen bisher nur direkt eingespritzt, was aber zur Folge hat, dass es keine Kontrolle über den Verbleib der Zellen im Defekt gibt. In der klinischen Anwendung bei der autologen Chondrocytentransplantation im Gelenk befinden sich bisher zwei Arten von Gelen: Kollagengele und Fibringele, wobei letztere durch eine enzymatisch katalysierte Reaktion gebildet werden. Dabei dient ersteres als Träger der Knorpelzellen, während Fibrin in erster Linie zur Abdichtung des reparierten Defektes nach Einbringung der Zellen mit oder ohne Träger verwendet wird.

Fibringele haben mehrere Nachteile. Sie können nachträglich stark aushärten und dadurch die Knorpelzellen schädigen. Ferner sind Fibringele teuer, da sie mit den rekombinanten Proteinen Thrombin und Faktor 13 hergestellt werden müssen. Ferner ist bei Fibringelen nachteilig, dass Fibrin selbst Entzündungsreaktionen auslösen kann, und ein Arbeiten mit den Gelen bei 37°C erfolgen muss.

Kollagengele werden im allgemeinen gut von den implantierten Zellen vertragen, sie haben aber den Nachteil, dass sie zum Teil - d.h. je nach Herstellungsart - beträchtlich schrumpfen können und daher tatsächlich Fehlschläge bei der Behandlung verursachen, da sie aus dem Defekt herausfallen können. Darüber hinaus gibt es auch neuere Befunde, dass Chondrocyten in Kollagengelen absterben können. Ferner sind Kollagengele wegen der hohen Viskosität nicht per Injektion applizierbar.

Insbesondere bei der Behandlung von Bandscheibenschäden besteht ein großes Bedürfnis an alternativen Behandlungsmethoden und an hierfür einzusetzende

Materialien. Bandscheiben verbinden die Wirbelkörper der Wirbelsäule miteinander und sorgen für die zur Beugung und Drehung notwendige Beweglichkeit und wirken im aufrechten Gang der durch Gravitation verursachten Kompression entgegen. Bandscheiben bestehen aus zwei Teilen, dem äußeren *Anulus fibrosus* (Faserring) und dem inneren *Nucleus pulposus* (Gallertkern). Der *Anulus fibrosus* besteht aus konzentrischen Schichten von kollagenen Bindegewebsfasern (Außenzone), die nach innen allmählich in Faserknorpel (Innenzone) übergehen. Der *Nucleus pulposus* (Bandscheibenkern) ist ein zellarmes gallertiges Gewebe mit einem hohen Wassergehalt. Er wirkt wie ein Wasserkissen stoßbrechend. Histologisch ist der gesunde Bandscheibenkern daher im Wesentlichen durch eine niedrige Zelldichte mit viel extrazellulärer Matrix ohne Blutversorgung, Lymphgefäße oder Innervierung charakterisiert. Die hiermit verbundene geringe intrinsische Regenerationskapazität führt zu einer erhöhten Degenerationsanfälligkeit der Bandscheibe. Da Bandscheibenkeme nicht vaskularisiert sind steht nach erlittenem Schaden in der Regel auch kein Zugang zu regenerativen Zellpopulationen zur Verfügung.

Standardbehandlungen sind gegenwärtig die Entfernung des Prolapses oder eine Nukleotomie, bzw. die Fusion der betroffenen Wirbelkörper. Bei beiden Methoden treten jedoch häufig schwere Folgen auf. Hierzu gehören das Post-Nukleotomie Syndrom und fortschreitende neurologische und klinische Symptome mit Auftreten einer Degeneration der Bandscheiben im angrenzenden Segment.

Obgleich Ätiologie und Pathophysiologie der Bandscheibendegeneration noch nicht vollständig geklärt sind, ist mittlerweile bekannt, dass eine Reihe verschiedener Prozesse die Alterung und Degeneration der Bandscheibe begleiten. So beeinflussen insbesondere die Zellvitalität und der Proteoglykangehalt direkt die Biomechanik der Bandscheibe, weshalb ein Zell- und Proteoglykanverlust eine der wichtigsten Ursachen für eine Bandscheibendegeneration darstellt.

Ein zu den Standardmethoden alternativer Ansatz stellt die Zelltransplantation dar. Versuche im Kaninchen- und im Hundemodell zeigten, dass die Behandlung einer degenerierten Bandscheibe mit autologen Bandscheibenzellen zu einer Revitalisierung des *Nucleus* führt.

Vor diesem Hintergrund ist es Aufgabe der vorliegenden Erfindung, eine Alternative zu den bisher im Stand der Technik verwendeten Ansätzen bereitzustellen, mit welcher die Nachteile der bekannten Verfahren und Materialien überwunden werden können.

Erfindungsgemäß wird diese Aufgabe gelöst durch eine injizierbare biokompatible Zusammensetzung basierend auf einem polymeren Träger, der zumindest ein hydrophiles Polymer aufweist, das *in situ* in einem Säuger polymerisierbar ist, wobei das zumindest ein hydrophile Polymer vernetzbares humanes Serumalbumin oder vernetzbares humanes Serum ist, wobei das humane Serumalbumin oder Serumpotein durch Gruppen funktionalisiert ist, die aus Maleimid-, Vinylsulfon-, Acrylat-, Alkylhalogenid-, Azirin-, Pyridyl-, Thionitrobenzolsäuregruppen, oder arylierenden Gruppen ausgewählt sind.

Die der Erfindung zu Grunde liegende Aufgabe wird auf diese Weise vollkommen gelöst.

Mit der erfindungsgemäßen Zusammensetzung wird ein Material bereitgestellt, das als eine biokompatible und bioabbaubare Hydrogel-Matrix geeignet ist und in einer besonderen Ausführungsform bspw. mit entsprechenden Zusätzen wie biologisch oder pharmazeutisch wirksamen Substanzen oder Zellen beladen werden kann. Dabei ermöglicht das Material den eingebrachten Zusätzen oder Zellen die Bioverfügbarkeit bzw. Lebensfähigkeit nach Einbringen an die gewünschte Stelle im Patienten, wobei das Material gleichzeitig *in situ* polymerisieren kann, ohne ev. darin vorliegende Zusätze wie lebende Zellen oder biologische oder pharmazeutisch aktive Agentien in ihrer Funktion oder Verfügbarkeit zu beeinträchtigen.

Die Erfinder haben nämlich in eigenen Versuchen erkannt, dass Serumalbumin bzw. Serumproteine als Trägermaterial für die Zwecke einer Verwendung zur Behandlung von Knorpel- oder Bandscheibendefekten/-degenerationen hervorragend geeignet sind. Dabei wird ausgenützt, dass Serumalbumin aus Blutserum und Lymphe von Säugern eine Trägersubstanz für körpereigene Substanzen ist. Serumalbumine sind in der Lage, eine große Anzahl unterschiedlicher Substanzen, wie z.B. Metallionen (Metalle), Fettsäuren und Aminosäuren, verschiedene Proteine und Arzneimittel zu binden, weshalb sie äußerst biokompatibel sind und so gut wie keine Reaktionen im Körper hervorrufen. Ferner ist bekannt, dass durch Albumin keine molekularen Reaktionen mit Knorpelzellen hervorgerufen werden.

Vorteilhaft bei der erfindungsgemäßen Zusammensetzung ist ferner, dass die Gelvorstufe bei Raumtemperatur gehandhabt werden kann. Das Material kann demnach separat von den jeweils einzubringen Zusätzen oder Zellen gelagert werden und kurz vor der Injektion mit den Zusätzen oder Zellen zusammengeführt werden. Die Polymerisationszeit ist dabei einstellbar, wobei Zeiten zwischen wenigen Sekunden und 2 Minuten vorgesehen sein können. Daher werden die Zusätze und/oder Zellen sofort in dem Material verankert, so dass ein unerwünschtes Diffundieren aus dem Material vermieden wird.

In der vorliegenden Anmeldung werden die Begriffe "Zusammensetzung" und "Material" für den beanspruchten Gegenstand verwendet, wobei "Zusammensetzung" überwiegend, aber nicht ausschließlich, für das noch nicht polymerisierte Material verwendet wird, und "Material" für die polymerisierte Zusammensetzung. Gleichwohl versteht es sich, dass diese Begriffe nicht vollständig voneinander getrennt werden können, da die Zusammensetzung und das Material faktisch den gleichen Gegenstand meinen.

Vorteilhaft ist ferner, dass der Grundstoff für das hydrophile Polymer variabel ist, so dass einerseits kommerziell verfügbares humanes Albumin, gereinigt oder rekombinant hergestellt, eingesetzt werden kann, sowie auch allogenes oder autologes Serum. Erste Versuche der Erfinder haben gezeigt, dass sich vernetztes Albumin nach ca. 14 Tagen auflöst. Dies ist von großem Vorteil, da innerhalb dieser Zeit bspw. Zellen eine perizelluläre Matrix entwickelt haben, und so in die Umgebung eingenistet sind. Damit wird mit der vorliegenden Erfindung ein Material bzw. eine Zusammensetzung bereitgestellt, die den Vorteil hat, dass sie während der Injektion flüssig ist und an der zu behandelnden und zu injizierenden Stelle in einem Patienten, bspw. in der Bandscheibe, zu einem Gel bzw. Hydrogel härtet, welches z.B. in der Zusammensetzung vorliegende Zellen daran hindert, aus dem Injektionsloch wieder auszutreten. Darüber hinaus ist das Material vorteilhafterweise bioabbaubar.

Dabei ist in einer Ausführungsform vorgesehen, wenn die Albuminkonzentration im fertigen, d.h. polymerisierten Gel von zwischen ca. 5 bis ca. 20, insbesondere ca. 10 mg/ml Gel beträgt.

Erfindungsgemäß ist in einer bevorzugten Ausführungsform vorgesehen, dass bspw. lebende Säugetierzellen, insbesondere humane lebende Zellen, sowie ein pharmakologisches Agens, ein biologisch wirksames Agens, oder eins oder mehrere oder Mischungen davon in der Zusammensetzung vorliegen.

Unter Säugetierzellen wird dabei jede Zelle verstanden, die von einem Säugetier abgeleitet ist bzw. abstammt, wobei hierunter insbesondere humane und tierische Zellen fallen. Solche Zellen können bspw. ausgewählt sein unter muskuloskelettäre Zellen, insbesondere Chondrocyten, Osteocyten, Fibrochondrocyten, sowie stoffwechselregulierende Drüsenzellen, Inselzellen, Melatonin-produzierende Zellen, Vorläuferzellen und Stammzellen, insbesondere mesenchymalen Stammzellen, mithin also Zellen, die für den jeweiligen Einsatz der Zusammensetzung, bzw. für die jeweilige Injektionsstelle geeignet und gewünscht sind.

Die Erfinder konnten in eigenen Studien zeigen, dass sowohl Chondrozyten als auch mesenchymale Stammzellen, die in der Zusammensetzung zur Differenzierung in Chondrozyten gebracht werden konnten, in der Zusammensetzung lebensfähig waren. Damit können solche erfindungsgemäßen Zusammensetzungen, die diese Zellen aufweisen, bspw. zur Regeneration von degeneriertem Knorpelgewebe und Bandscheiben vorteilhaft eingesetzt werden.

Insbesondere mesenchymale Stammzellen haben den Vorteil, dass durch den Rückgriff auf diese Zellen auch Fälle behandelt werden können, in denen kein autologes Bandscheibengewebe als Zellquelle zur Verfügung steht. Obgleich in einer Ausführungsform bevorzugt ist, wenn autologes Zellmaterial erfindungsgemäß in der Zusammensetzung eingesetzt wird, können auch Spenderzellen, insbesondere mesenchymale Stammzellen eingesetzt werden. In eigenen Studien konnten die Erfinder zeigen, dass mesenchymale Stammzellen (im Folgenden auch "MSC") in ausreichender Menge und guter Qualität aus allen getesteten Spendern (über 100) isoliert werden konnten. Damit stellen die adulten mesenchymalen Stammzellen eine nahezu beliebig expandierbare Zellquelle dar, die über eine Punktion des Beckenkamms und Aspiration von Knochenmark für den Operateur problemlos zugänglich ist, und die *in vitro* in mehrere Zelltypen der mesenchymalen Linie differenziert werden kann, eingeschlossen der chondrogenen.

Für die Zwecke der vorliegenden Erfindung können daher bspw. entweder autologe oder allogene mesenchymale Stammzellen isoliert, expandiert und bspw. zu Chondrozyten differenziert werden, sowie anschließend in die Zusammensetzung eingebracht werden. Auf der anderen Seite ist es aber auch möglich, die mesenchymalen Stammzellen direkt in die Zusammensetzung einzubringen, bspw. zusammen mit entsprechenden Differenzierungsfaktoren, die dann *in situ* eine entsprechende Differenzierung des Stammzellen bewirken.

Die erfindungsgemäße Zusammensetzung, die mit den gewünschten und jeweils für den spezifischen Einsatz in Frage kommenden Zellen beladen ist, hat den Vorteil, dass mit ihr eine Unterstützung des Phänotyps des zu regenerierenden Gewebes erreicht werden kann. Daneben kann auch eine *de novo* Synthese und die Ablage extrazellulärer Matrix gefördert werden, was insbesondere für die Behandlung von Bandscheibendefekten wünschenswert ist.

Daneben können die Zusammensetzungen auch als eine Art temporäre Zelldepots eingesetzt und hierfür bspw. mit Zellen beladen werden, die bestimmte Hormone produzieren, wie bspw. Insulin, Thyroxin oder Melatonin. Die in der Zusammensetzung vorliegenden Zellen werden an die gewünschte Stelle injiziert, wonach die Zusammensetzung *in situ* polymerisiert. Die in der Zusammensetzung vorliegenden Zellen sind in dem polymerisierten Material lebendfähig, produzieren die jeweiligen Hormone und setzen diese an die Umgebung frei.

Ferner kann in einer anderen Ausführungsform vorgesehen sein, dass mit der Zusammensetzung entsprechende Zellen in große Wundgebiete eingebracht werden, bspw. der Haut, Leber oder Milz, um dort eine Regeneration des verwundeten Gewebes zu bewirken.

Die Zusammensetzung kann anstelle von oder neben Zellen auch eine oder mehrere biologisch oder pharmazeutisch aktive Substanzen, oder Mischungen, davon aufweisen.

"Biologisch aktive bzw. wirksame Substanz" und "pharmazeutisch aktive bzw. wirksame Substanz" soll vorliegend jede natürliche oder synthetische Substanz bedeuten, die entweder einen biologischen oder pharmazeutischen Einfluss auf Zellen oder Gewebe haben kann, bzw. die Reaktionen auf oder in Zellen ausüben kann. Dieser Einfluss kann dabei auf bestimmte Zellen und bestimmte Bedingungen beschränkt sein, ohne dass die Substanz ihre biologisch oder pharmazeutische aktive Bedeutung verlieren würde. Die chemische Beschaffenheit der vorliegend verwendbaren Substanzen ist dabei nicht auf eine bestimmte (Verbindungs-)Klasse beschränkt, sondern kann vielmehr jede natürliche und synthetische Substanz mit einschließen, die von ihrer Natur aus und/oder in modifizierter Form irgendeine Wirkung auf biologische Zellen ausübt.

So ist insbesondere bevorzugt, wenn als biologisch oder pharmazeutisch aktive bzw. wirksame Substanzen bspw. Antibiotika, entzündungshemmende Mittel, Stoffwechsel-Hormone, Chondroprotektiva, Agentien zur Gentherapie, Wachstumshormone oder Differenzierungs- und/oder Modulationsfaktoren, Immunsuppresiva, immun-stimulierende Substanzen, allgemein Peptide, Proteine, Nukleinsäuren, organische Wirkstoffe, Hyaluronsäure, Apoptose-induzierende Wirkstoffe, adhäsionsvermittelnde Wirkstoffe, Rezeptoragonisten- und Rezeptorantagonisten, oder Mischungenö davon, eingesetzt werden. Ferner können Proteine der extrazellulären Matrix, Proteine der Zelloberfläche, sowie allgemein Polysaccharide, Lipide, Antikörper, Wachstumsfaktoren, Zucker, Lektine, Kohlenhydrate, Zytokine, DNA, RNA, siRNA, Aptamere, sowie bindungs- oder wirkungsrelevante Fragmente davon, sowie sogenannte Disease-modifiying Osteoarthritis Agents, oder Mischungen davon, eingesetzt werden. Dabei können sämtliche Substanzen synthetisch hergestellt oder natürlich vorkommend sein bzw. aus rekombinanten Quellen stammen. Unter "Diseasemodifying Osteoarthritis Agents" (DMOAs) sind dabei eine Reihe von Substanzen zu verstehen, die als Medikament gegenwärtig insbesondere bei Arthrose - inzwischen aber auch bei weiteren Autoimmunkrankheiten - zur Linderung von Schmerzen und Entzündungen eingesetzt werden, und deren exakter Wirkungsmechanismus bisher noch nicht umfassend geklärt ist. Die meisten dieser Substanzen enthalten Mischungen aus Glucosamin und Chondroitin-Sulfat.

Insbesondere ist in einer Ausführungsform bevorzugt, wenn die biologisch wirksame Substanz Hyaluronsäure ist und in dem Gel in einer Endkonzentration von zwischen ca. 1 bis ca. 10 mg/ml Gel vorliegt, insbesondere mit a. 4 mg/ml Gel.

Unter "bindungs- oder wirkungsrelevanten Fragmenten davon" werden Teile bzw. Abschnitte der aufgeführten Substanzen gemeint sind, die, obwohl ev. nicht die komplette Substanz eingesetzt wird, für sich genommen immer noch die gleiche bzw. nahezu gleiche oder zumindest ähnliche Reaktion oder Wirkung auf Zellen ausüben, wie die komplette Substanz. Unter Reaktion kann dabei bereits die bloße Bindung von/an Zellen zu verstehen sein, aber auch die einer Bindung anschließende Reaktion in einer Zelle auf die Bindung, wie bspw. die Auslösung von bestimmten Reaktionswegen in den Zellen, die zu einer Produktion/Ausschüttung bestimmter Substanzen durch die Zellen führen können, oder aber zur einer Umwandlung oder Differenzierung der Zellen.

Weitere Beispiele schließen, jedoch nicht ausschließlich, die folgenden synthetischen oder natürlichen oder rekombinanten Quellen davon mit ein: Wachstumshormone, einschließlich humanem Wachstumshormon und rekombinatem Wachstumshormon (rhGH), Rinderwachstumshormone, Schweinewachstumshormone; Wachstumshormon-freisetzende Hormone; Interferone, einschließlich Interferon-alpha, - beta und -gamma; Interleukin-1; Interleukin-2; Insulin; Insulin-ähnlicher Wachstumsfaktor, einschließlich IGF-1; Heparin; Erythropoietin; Somatostatin; Somatotropin; Proteaseinhibitoren; Adrenocorticotropin; Prostaglandine; sowie Analoga, Fragmente, Mimetika oder Polyethyleneglycol(PEG)-modifizierte Derivate dieser Verbindungen; oder eine Kombination davon. Es versteht sich, dass alle gegenwärtig im allgemeinen Gebiet der Therapie von Erkrankungen mit von Trägern/Matrizes *in situ* freizusetzenden (Wirk-)stoffen zur Anwendung für die vorliegende Erfindung in Frage kommen, wobei dem Fachmann jeweils klar sein wird, dass der einzusetzende (Wirk-)Stoff bzw. die einzusetzenden Zellen vom jeweiligen zu behandelnden Fall abhängen.

Gemäß der erfindungsgemäßen Zusammensetzung ist das humane Serumalbumin oder das humane Serumprotein durch Gruppen funktionalisiert, die aus Maleimid-, Vinylsulfon-, Acrylat-, Alkylhalogenid-, Azirin-, Pyridyl-, Thionitrobenzolsäuregruppen, oder arylierenden Gruppen ausgewählt sind.

Unter "funktionalisiert" bzw. funktionalisieren ist vorliegend jeder - abgeschlossene - Vorgang zu verstehen, mit dem dem Polymer - bspw. durch Hinzufügen von Gruppen an das Polymer - eine Funktion verliehen wird, die es normalerweise nicht besitzt.

Die Erfinder konnten in eigenen Versuchen zeigen, dass durch die Funktionalisierung des Polymers mit Maleimidgruppen eine gute Vernetzung des Polymers und gleichzeitig die Lebensfähigkeit von Zellen oder Biofunktionalität von Substanzen erreicht und gewährleistet werden kann.

Die in die Zusammensetzung ggf. einzubringenden Zellen oder Substanzen werden durch Dispergierung in die Zusammensetzung mit dem funktionalisierten Polymer eingebracht, das mit dem Zellen vernetzt.

Der Einsatz von funktionalisiertem humanem Serumalbumin bzw. Serumprotein als injizierbare, *in situ* polymerisierbare Zusammensetzung ist im Stand der Technik weder offenbart noch nahe gelegt.

Die Erfindung betrifft ferner die Verwendung von funktionalisiertem humanem Serumalbumin und/oder Serumprotein zur Herstellung einer injizierbaren biokompatiblen Zusammensetzung, insbesondere die Verwendung von autologem Serumalbumin und/oder Serumprotein.

Mit der vorliegenden Verwendung wird vorteilhaft die Bereitstellung einer Zusammensetzung ermöglicht, die hervorragend als injizierbares, *in situ* polymerisierbares Material geeignet ist, in das darüber hinaus auch bspw. Zellen und/oder biologisch bzw. pharmazeutisch aktive Substanzen aufgenommen werden können. Die Zusammensetzung gewährleistet nämlich die Überlebensfähigkeit und die Bioverfügbarkeit der darin dispergierten Zusätze, und ist darüber hinaus für den zu behandelnden Patienten völlig unbedenklich und gut verträglich, da die Grundsubstanz bzw. das hydrophile Polymer, nämlich Albumin oder Serumprotein, eine Substanz darstellt, die regelmäßig in Körperflüssigkeiten zu finden ist. Nach der Injektion in einen Patienten an die zu behandelnde Stelle polymerisiert die Zusammensetzung; dadurch kann das Material das betreffende, zu behandelnde Gewebe einerseits mechanisch unterstützen, und/oder durch die in der Zusammensetzung vorliegenden Zellen und/oder Substanzen das Gewebe regenerieren.

Dabei ist insbesondere bevorzugt, wenn maleimidfunktionalisiertes humanes Serumalbumin oder maleimidfunktionalisiertes humanes Serumprotein eingesetzt wird. Erfindungsgemäß ist dabei vorgesehen, dass die Zusammensetzung zur Herstellung biokompatibler abbaubarer Materialien zur Verwendung in der Pharmakologie, Veterinär- und/oder Humanmedizin eingesetzt wird.

Wie weiter oben erläutert, eignet sich die Zusammensetzung bzw. vielmehr das hieraus hergestellte biokompatible Material als Bestandteil oder als Ganzes in implantierbaren Vorrichtungen bzw. als Implantat zur Verwendung bei der Wiederherstellung, der Rekonstruktion und/oder dem Ersatz von Geweben und/oder Organen, oder als Drug-Release-Implantat bei Säugetieren.

Dabei ist insbesondere bevorzugt, wenn die Zusammensetzung zum Ersatz und/oder zur Regeneration von Gelenkknorpeln, Bandscheiben, Knochengewebe, gewebeaufbauenden Zellen, und stoffwechselregulierender sekretorischer Zellen eingesetzt wird.

Die Erfinder konnten in eigenen Studien zeigen, dass die erfindungsgemäße Zusammensetzung, beladen mit Zellen, und nach Injizierung und *in situ* Polymerisierung keine Gewebeunverträglichkeiten hervorgerufen wurden. Gleichzeitig waren die in der Zusammensetzung vorliegenden Zellen vital. Dies zeigt, dass mit der vorliegenden Zusammensetzung ein hervorragendes Biomaterial bereitgestellt wird, mit dem degeneriertes Gewebe durch Bereitstellen von Zellen wieder regeneriert werden kann.

Es versteht sich, dass je nach zu regenerierendem Gewebe entsprechende Zellen und/oder Zusätze in die Zusammensetzung gegeben werden. So können bspw. bei einer Behandlung von Bandscheibenschäden Chondrozyten oder mesenchymale Stammzellen eingesetzt werden, die die degenerierte Bandscheibe regenerieren können. Andererseits kann die Zusammensetzung bspw. auch als so genanntes Drug-Release-System eingesetzt werden, wobei in diesem Fall die Zusammensetzung mit dem gewünschten und vorgesehenen Wirkstoff/Arzneimittel/Pharmazeutikum beladen wird und in den Patienten injiziert wird. Nach Polymerisierung der Zusammensetzung im Patienten kann der Wirkstoff aus der polymerisierten Zusammensetzung freigesetzt werden, da diese bioabbaubar ist. Vorteilhaft ist hierbei, dass eine lokale Verabreichung stattfinden kann, so dass den Körper belastende systemische Verabreichungen vermieden werden können. Dem Fachmann wird klar sein, ob und welche Zellen oder Zusätze bzw. biologisch oder pharmazeutisch aktiven Substanzen der Zusammensetzung hinzugefügt werden können bzw. müssen, um einen optimalen Behandlungsansatz zu gewährleisten.

Die Erfindung betrifft ferner ein Verfahren zur Herstellung einer injizierbaren biokompatiblen Zusammensetzung, das die folgenden Schritte aufweist:
a) Bereitstellen von humanem Serumprotein oder Serumalbumin;
b) funktionalisieren mit Maleimid, Vinylsulfon, Acrylat, Alkylhalogenid, Azirin, Pyridyl, Thionitrobenzolsäure, oder arylierenden Gruppen;
c) ggf. Hinzufügen von Säugetierzellen und/oder biologisch oder pharmazeutische aktiven Substanzen; und
d) Hinzufügen eines Vernetzers zum Vernetzen des in Schritt b) funktionaliserten humanen Serums oder Serumalbumins.

Als Vernetzer kommen dabei bspw. Substanzen in Betracht, die SH-Gruppen tragen, insbesondere Polymere, und bspw. Dithio-PEG oder SH-modifiziertes Dextran, SH-modifiziertes Polyvinylalkohol, SH-modifiziertes Polyvinylpyrrolidon oder SH-modifiziertes Polyethylenglykol (SH-PEG).

So beruht die Reaktionschemie des erfindungsgemäßen Verfahrens bspw. in einer bevorzugten Ausführungsform auf der Verwendung von Maleindiimid als divalentes Konjugationsmolekül, das einseitig an Albumin gekoppelt wird. Bei der eigentlichen Vernetzungsreaktion reagiert die zweite Seite des Albumin mit einem doppelseitig thiolierten Polyethylenglykol (SH-PEG), das als Koppler zwischen zwei am Albumin hängenden Maleinimiden fungiert. Das SH-PEG dient dabei auch als Abstandshalter, um genügend Zwischenräume für Wasser und diffundierende kleine Moleküle zu schaffen. In der Vernetzungsreaktion kann dann auch bspw. Hyaluronsäure vorliegen, deren sehr lange Ketten durch die Vernetzungsreaktion im Gel eingefangen werden. Dadurch entsteht eine physikalisch stabile Gelkonformation, bei der die Hyaluronsäure nicht mehr durch Diffusion aus dem Gel entweichen kann. Erst durch den biologischen Abbau des Gels *in situ* führt zur Freisetzung von Abbauprodukten wie u.a. und insbesondere Maleat (Äpfelsäure) - und bei Vorliegen von Hyaluronsäure - von Hyaluronsäurefragmenten.

Zur Herstellung einer Ausführungsform des erfindungsgemäßen Gels wird also eine Albumin/Hyaluronsäure/Zellsuspension enthaltende Zusammensetzung mit dem Vernetzer unmittelbar vor Einbringen in die zu behandelnde Stelle in Kontakt gebracht. Hierzu werden die aktiven Komponenten (Albumin/ggf. Hyaluronsäure/Zellsuspension mit bspw. 2 ml und bspw. SH-PEG-Lösung mit bspw. 0,5 ml in eine Doppelkammerspritze eingebracht, und durch Hereinschieben des Kolbens durch eine Mischkammer geschickt. Die Polymerisationsreaktion erfolgt dann in ca. 2 bis 3 Minuten. Dadurch verbleibt vorteilhafterweise genügend Zeit für eine gleichmäßige Verteilung der Gelmischung im Gewebe.

Weitere Vorteile ergeben sich aus der Beschreibung und der beigefügten Zeichnung.
- Fig. 1: Ein Diagramm zur Analyse der Expression bestimmter Markergene nach Induktion der chondrogenen Differenzierung von humanen Stammzellen; und
- Fig. 2a, b: Giemsa-gefärbte Schweinechondrocyten im Albumingel.

### Beispiel

### Herstellung von maleimidmodifiziertem Rinderserumalbumin

250 mg humanes Serumalbumin (Sigma-Aldrich, Kat.-Nr. A1653) wurden in 5 ml 1M Na-Borat (pH 8,2) gelöst. Dazu wurden 75 µl einer 260 mM N-Maleoyl-β-Alanin (Sigma-Aldrich Kat.-Nr 63285)-Lösung in PBS/na-Borat (pH 8,2) (1:1) hinzugefügt, und für 90 min bei Raumtemperatur inkubiert. 106 mg 3-Maleimidopropionsäure-N-Hydroxysuccinimidester (SMP, Obiter Research, Urbana, IL, USA) in 950 µl Dimehtylformamid (DMF) gelöst. Unlösliches Material wurde durch Zentrifugation abgetrennt. 500 µl des Überstandes wurden zu der Albuminlösung gegeben, die danach für 60 min bei Raumtemperatur inkubiert wurde. Danach wurden 500 µl 3M Natriumacetat (pH 4,7) dazu gegeben und dreimal gegen 1 Liter PBS auf Eis dialysiert. Das Dialysat wurde anschließend durch Ultrafiltration (YM-3 Membran, Millipore) auf ein Volumen von 3,5 ml konzentriert, filtersterilisiert und bei -80°C gelagert.

### Herstellung von maleimidmodifiziertem humanem Serum

Zu 5 µl humanem Serum (AB-Pool, Tetec GmbH, Reutlingen) wurden 75 µl einer 260 mM N-Maleoyl-β-Alanin (Sigma Kat.-Nr 63285)-Lösung in PBS/1 M Na-Borat (pH 8,2) (1:1) gegeben und für 90 min bei Raumtemperatur inkubiert. Danach wurden 0,5 ml 1M Na-Borat (pH 8,2) dazu gegeben. Dazu wurden 500 µl des SMP-Überstands (Beispiel 1) gegeben, und für 60 min bei Raumtemperatur inkubiert. Danach wurden 500 µl 3 M Natriumacetat (pH 4,7) dazu gegeben und dreimal gegen 1 Liter PBS auf Eis dialysiert. Das Dialysat wurde durch Ulrafiltration (YM-3-Membran, Millipore) auf 3,5 ml konzentriert, Unlösliches Material wurde durch eine Passage durch den Stopfen einer 1 ml Pipettenspitze abgetrennt. Das Filtrat wurde filtersterilisiert und bei -80°C gelagert.

### Herstellung von maleimidmodifiziertem Rinderserumalbumin

250 mg BSA (Sigma-Aldrich, Kat.-Nr. A7030) wurden in 5 ml 1M Na-Borat (pH 8,2) gelöst. Diese Lösung wurde mit 6 mg N-Maleoyl-β-Alanin (Sigma-Aldrich Kat.-Nr 63285) versetzt und für 2 Stunden bei Raumtemperatur inkubiert. Weiterhin wurden 130 mg N-Hydroxysuccinimid in 730 µl Acetonitril gelöst. Danach wurden 96 mg N-Maleoyl-β-Alanin in 570 µl der N-Hydroxysuccinimid-Lösung gelöst. Zu dieser Lösung wurden 80 µl Acetonitril und 80 µl Diisopropylcarbodiimid gegeben. Nach einer 5-minütigen Inkubation bei Raumtemperatur wurde der Ansatz für 5 min zentrifugiert. Der Überstand wurde tropfenweise zu der BSA-Lösung unter ständigem Rühren zugegeben. Nach Inkubation von 45 min bei Raumtemperatur wurde der Ansatz viermal gegen 500 ml PBS auf Eis dialysiert und anschließend durch Ultrafiltration auf ein Volumen von 4 ml konzentriert.

### Test der Albumingele als Träger für Chondrozyten in vitro

### Durchgeführte Experimente - Zellkultur:

Chondrogene Differenzierung von humanen, mesenchymalen Stammzellen, in humanen Serum- und Serumalbumin-Gelen im Vergleich zur Standardmethode (Spheroidkultur)

### Durchführung:

Für die Herstellung der Gele oder Spheroide wurden humane mesenchymale Stammzellen (P2 oder P3) verwendet.

### Herstellung Gele:

Die MSCs wurden in Konzentrationen von 1 Mio. bzw. 5 Mio. pro ml Gel wie folgt eingebettet:

| | Humanes Serumalbumin | Humanes Serum |
|---|---|---|
| SPS in Platte vorlegen | 34 µl | 24 µl |
| Zellpellet | 1 Mio. | 1 Mio. |
| mit Medium resuspendieren | 83 ul | 88 µl |
| mit dem Gel-Material mischen und in die | 83 µl | 88 µl |
| Endvolumen | 200 µl | 200µl |

Nach 5 Minuten Polymerisation bei Raumtemperatur wurden die Gele mit 500 µl chondrogenem Induktionsmedium überschichtet.

### Herstellung Spheroide:

0,5 Mio. Zellen wurden in 20 µl Medium aufgenommen und vorsichtig als einzelner Tropfen in eine Wellplatte pipettiert, anschließend für 2 - 4 h inkubiert und dann mit chondrogenem Induktionsmedium überschichtet (500 µl).

### Kultivierungsdauer: 14-21 Tage

Die Auswertung der unterschiedlichen Ansätze erfolgte durch eine Genexpressionsanalyse im Vergleich zu den Ausgangszellen vor der Induktion.

### Ergebnis:

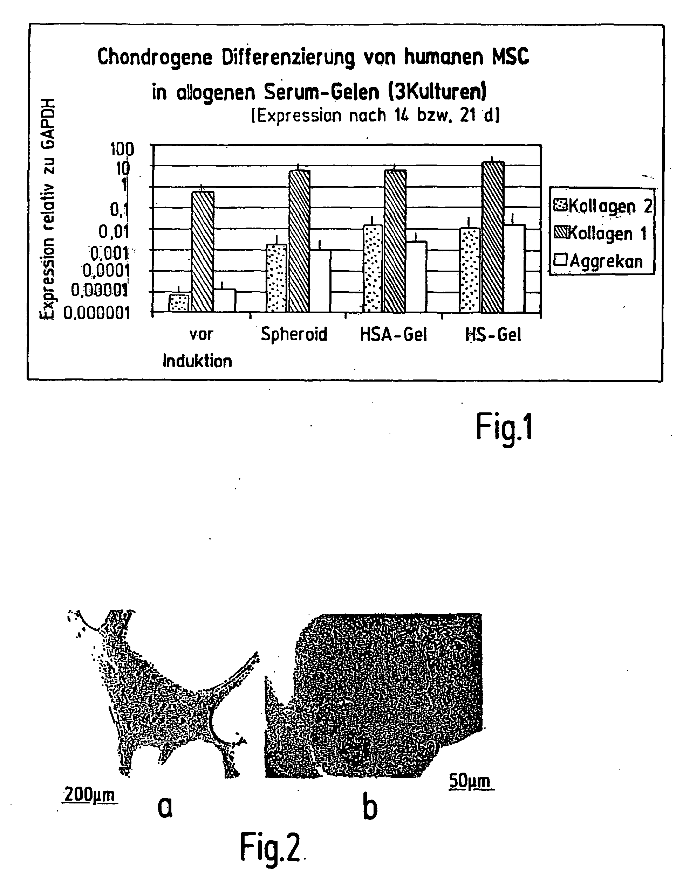

In beiden humanen Gel-Materialien konnte eine der Spheroid-Kultur entsprechende chondrogene Differenzierung der Stammzellen induziert werden (siehe hierzu das Diagramm in Fig. 1). Untersucht wurde die Genexpression von Kollagen-1 (graue Balken), Kollagen-2 (schwarze Balken) und Aggrekan (weiße Balken), 14 bzw. 21 Tage nach Induktion. Als Maßstab diente dabei die Expression von GAPDH (Glycerinaldehyd-3-phosphat-Dehydrogenase).

Wie dem Diagramm zu entnehmen ist, zeigte die Analyse der Expression der genannten Markergene nach Induktion der chondrogenen Differenzierung von humanen Stammzellen, dass in den HSA (humanes Serumalbmin) - bzw. HS (humanes Serum) - Gelen die Expression von Kollagen-2 und Aggrekan deutlich erhöht wird. Damit ist sie mit der Genexpression im Spheroid-Modell vergleichbar. (Quantitative rt-PCR, Expression normiert auf GAPDH).

Eine Beobachtung des Differenzierungsprozesses über einen längeren Zeitraum (> 14 d) war nicht möglich, da die humanen HS- und HSA-Gele unter Induktionsbedingungen nach 10-14 Tagen in Kultur nahezu vollständig abgebaut waren.

**Test der Albumingele als Träger für Chondrozyten *in vivo***

### Versuchsdurchführung:

### 1) Chondrozyten (Sus scrofa) und Gel (Zelllabor)

Vor Injektion des Gels in die Scid-Maus wurde im Zelllabor eine Mischung aus BSA7-Gel und den verwendeten porcinen Chondrozyten vorbereitet.

Dazu wurden in einem Eppendorf-Gefäß 1 Million porcine Chondrozyten abzentrifugiert und das Pellet in 400 µl DMEM resuspendiert. Anschließend wurde 400ul BSA7-Gel zugegeben und die Lösung durch zweimaliges hoch- u. runterpipettieren gemischt. Das Gel wurde zum Transport auf Eis gelagert. 120µl SPS-Vemetzer wurden in ein seperates Eppendorf-Cup überführt und ebenfalls auf Eis gelagert.

### 2) Mausversuch (Tierstall)

Im Tierstall wurden die beiden Komponenten direkt vor der Injektion in die Maus gemischt. Dazu wurden die 120 µl SPS-Vernetzer zunächst über eine 0,6 mm - Kanüle in eine 5 ml-Spritze gezogen und eine kleine Luftkammer erzeugt. Dann wurde die vorbereitete 800 µl Gel-Zell-Mischung durch eine 0,9 mm-Kanüle aufgezogen und somit mit dem Vernetzer vermischt. Die Injektion erfolgte direkt im Anschluss subcutan unter die Nackenhaut der mit Ketanest narkotisierten SCID-Mäuse (2 Tiere). Beim Injizieren bildete sich ein kugelförmiges Gel.

Die Mäuse wurden 10 Tage normal gehalten, dann getötet. Die Rückenhaut wurde durch einen Schnitt eröffnet und die Gele freipräpariert. Es waren makroskopisch keine Anzeichen von Gewebeunverträglichkeit, wie Entzündungen und/oder Eiter, festzustellen.

Die Gelstücke wurden dann entfernt und in 10%-iger Formalinlösung über Nacht bei 4°C fixiert, danach in 1 %-iger Formalinlösung bei 4°C bis zur Verwendung in der Histologie gelagert. Zur Histologie wurden Gefrierschnitte von ca. 50 um Dicke angefertigt und frisch angefärbt (Giemsa, DAPI). Die nassen Schnitte wurden direkt mit einem Deckglas bedeckt und unmittelbar danach mikroskopiert (siehe Fig. 2). Die Gele waren schaumig porös organisiert. Alle auffindbaren Zellen waren vital. Die Chondrozyten waren zum Teil in Proliferation befindlich und von einem Hof von extrazellulärer Matrix umgeben. An der Peripherie der Implantate waren zum Teil fibroblastoide kleinere Zellen auffindbar, wahrscheinlich eingewanderte Bindegewebezellen aus der Maus.

## Patentansprüche

1. Injizierbare biokompatible Zusammensetzung, basierend auf einem polymeren Träger, der zumindest ein hydrophiles Polymer aufweist, wobei das Polymer *in situ* zu einem Gel polymerisierbar ist, **dadurch gekennzeichnet, dass** das hydrophile Polymer vernetzbares humanes Serumalbumin oder vernetzbares humanes Serumprotein ist, und dass das humane Serumalbumin oder das humane Serumprotein durch Gruppen funktionalisiert ist, die aus Maleimid-, Vinylsulfon-, Acrylat-, Alkylhalogenid-, Azirin-, Pyridyl-, Thionitrobenzolsäuregruppen, oder arylierenden Gruppen ausgewählt sind.

2. Zusammensetzung nach Anspruch 1, **dadurch gekennzeichnet, dass** sie ferner zumindest eines der Folgenden aufweist, nämlich Säugerzellen, ein pharmakologisches Agens, ein biologisch wirksames Agens, oder eins oder mehrere oder Mischungen davon.

3. Zusammensetzung nach Anspruch 2, **dadurch gekennzeichnet, dass** die Säugerzellen ausgewählt sind aus muskuloskelettären Zellen, insbesondere Chondrocyten, Osteocyten, Fibrochondrocyten, sowie stoffwechselregulierenden Drüsenzellen, Inselzellen, Melatonin-produzierenden Zellen, Vorläuferzellen und Stammzellen, insbesondere mesenchymalen Stammzellen.

4. Zusammensetzung nach Anspruch 2, **dadurch gekennzeichnet, dass** das pharmakologisch wirksame Agens ausgewählt ist aus zumindest einem der Folgenden: einem Antibiotikum, einem entzündungshemmendem Mittel, einem Stoffwechsel-Hormon, Chondroprotektiva, Agentien zur Gentherapie, Wachstumshormone, Differenzierungs- oder Modulationsfaktoren, Immunsuppresiva, immun-stimulierende Substanzen, DMOAs, Nucleinsäuren, Apoptose-induzierende Wirkstoffe, adhäsionsvermittelnde Wirkstoffe, Rezeptoragonisten- und Rezeptorantagonisten, oder Mischungen davon.

5. Zusammensetzung nach einem der Ansprüche 1 bis 4, **dadurch gekennzeichnet, dass** die Albuminkonzentration in dem Gel von ca. 5 bis ca. 15 mg/ml Gel, insbesondere von ca. 10 mg/ml Gel beträgt.

6. Zusammensetzug nach einem der Ansprüche 1 bis 5, **dadurch gekennzeichnet, dass** das biologisch wirksame Agens Hyaluronsäure ist.

7. Zusammensetzung nach einem der Ansprüche 1 bis 6, **dadurch gekennzeichnet, dass** die Hyaluronsärekonzentration in dem Gel von ca. 1 bis ca. 10 mg/ml Gel, insbesondere ca. 4 mg/ml Gel beträgt.

8. Verwendung von funktionalisiertem humanem Serumalbumin oder funktionalisiertem humanem Serumprotein zur Herstellung einer injizierbaren biokompatiblen Zusammensetzung, wobei das humane Serumalbumin oder humane Serumportein durch Gruppen funktionalisiert ist, die aus Maleimid-, Vinylsulfon-, Acrylat-, Alkylhalogenid-, Azirin-, Pyridyl-, Thionitrobenzolsäuregruppen, oder arylierenden Gruppen ausgewählt sind.

9. Verwendung nach Anspruch 8, **dadurch gekennzeichnet, dass** maleimidfunktionalisiertes Serumalbumin oder maleimidfunktionalisiertes Serumprotein eingesetzt wird.

10. Verwendung einer Zusammensetzung nach einem der Ansprüche 1 bis 7 zur Herstellung biokompatibler abbaubarer Materialien zur Verwendung in der Pharmakologie, Veterinär- und/oder Humanmedizin.

11. Verwendung nach Anspruch 10, **dadurch gekennzeichnet, dass** bei der Verwendung die Zusammensetzung als Ganzes oder als Komponente in implantierbaren Vorrichtungen oder in Implantaten zur Verwendung bei der Wiederherstellung, der Rekonstruktion und/oder dem Ersatz von Geweben und/oder Organen, oder als Drug-Release-Implantat bei Säugetieren eingesetzt wird.

12. Verwendung nach Anspruch 11, **dadurch gekennzeichnet, dass** bei der Verwendung die Zusammensetzung zum Ersatz und/oder zur Regeneration von Gelenkknorpeln, Bandscheiben, Knochengewebe, gewebeaufbauenden Zellen, und stoffwechselregulierenden sekretorischer Zellen eingesetzt wird.

13. Verfahren zur Herstellung einer injizierbaren biokompatiblen Zusammensetzung mit zumindest einem hydrophilen Polymer, das *in situ* zu einem Gel polymerisierbar ist, welches die folgenden Schritte aufweist:
a) Bereitstellen von humanem Serum oder humanem Serumalbumin;
b) funktionalisieren mit Maleimid, Vinylsulfon, Acrylat, Alkylhalogenid, Azirin, Pyridyl, Thionitrobenzolsäure, oder Derivaten davon, oder arylierenden Gruppen;
c) ggf. Hinzufügen von Säugetierzellen, eines pharmakologischen Agens, eines biologisch wirksamen Agens, oder Mischungen davon; und
d) Hinzufügen eines Vernetzers zum Vernetzen des in Schritt b) funktionaliserten humanem Serumproteins oder humanem Serumalbumins.

## Claims

1. Injectable biocompatible composition based on a polymeric carrier which comprises at least one hydrophilic polymer, wherein the polymer is polymerizable *in situ* to form a gel, **characterized in that** the hydrophilic polymer is crosslinkable human serum albumin or crosslinkable human serum protein, and **in that** the human serum albumin or the human serum protein is functionalized by groups which are selected from maleimide, vinyl sulfone, acrylate, alkyl halide, azirine, pyridyl, thionitrobenzoic acid groups, or arylating groups.

2. The composition as claimed in claim 1, **characterized in that** it further comprises at least one of the following, namely mammalian cells, a pharmacological agent, a biologically active agent, or one or more or mixtures thereof.

3. The composition as claimed in claim 2, **characterized in that** the mammalian cells are selected from skeletal muscle cells, in particular chondrocytes, osteocytes, fibrochondrocytes, and also metabolism-regulating glandular cells, islet cells, melatonin-producing cells, progenitor cells and stem cells, in particular mesenchymal stem cells.

4. The composition as claimed in claim 2, **characterized in that** the pharmacologically active agent is selected from at least one of the following: an antibiotic, an anti-inflammatory, a metabolism hormone, chondroprotectives, agents for gene therapy, growth hormones, differentiation or modulation factors, immunosuppressives, immunostimulatory substances, DMOAs, nucleic acids, apoptosis-inducing actives, adhesion-mediating actives, receptor agonists and receptor antagonists, or mixtures thereof.

5. The composition as claimed in any of claims 1 to 4, **characterized in that** the albumin concentration in the gel is from about 5 to about 15 mg/ml gel, in particular from about 10 mg/ml gel.

6. The composition as claimed in any of claims 1 to 5, **characterized in that** the biologically active agent is hyaluronic acid.

7. The composition as claimed in any of claims 1 to 6, **characterized in that** the hyaluronic acid concentration in the gel is from about 1 to about 10 mg/ml gel, in particular about 4 mg/ml gel.

8. Use of functionalized human serum albumin or functionalized human serum protein for preparing an injectable biocompatible composition, wherein the human the serum albumin or human serum protein is functionalized by groups which are selected from maleimide, vinyl sulfone, acrylate, alkyl halide, azirine, pyridyl, thionitrobenzoic acid groups, or arylating groups.

9. The use as claimed in claim 8, **characterized in that** maleimide-functionalized serum albumin or maleimide-functionalized serum protein is used.

10. The use of a composition as claimed in any of claims 1 to 7 for preparing biocompatible degradable materials for use in pharmacology, veterinary and/or human medicine.

11. The use as claimed in claim 10, **characterized in that**, in the use, the composition is used as a whole or as a component in implantable devices or in implants for use in the restoration, the reconstruction, and/or the replacement of tissues and/or organs, or as a drug release implant in mammals.

12. The use as claimed in claim 11, **characterized in that**, in the use, the composition is used for replacing and/or for regenerating articular cartilage, intervertebral discs, bone tissue, tissue-building cells, and metabolism-regulating secretory cells.

13. A process for preparing an injectable biocompatible composition having at least one hydrophilic polymer which is polymerizable *in situ* to form a gel, comprising the following steps:
a) providing human serum or human serum albumin;
b) functionalizing with maleimide, vinyl sulfone, acrylate, alkyl halide, azirine, pyridyl, thionitrobenzoic acid, or derivatives thereof, or arylating groups;
c) optionally adding mammalian cells, a pharmacological agent, a biologically active agent, or mixtures thereof; and
d) adding a crosslinker for crosslinking the human serum protein or human serum albumin functionalized in step b).

## Revendications

1. Composition biocompatible pouvant être injectée, à base d'un support polymère, qui présente au moins un polymère hydrophile, sachant que le polymère peut être polymérisé in situ en un gel, **caractérisée en ce que** le polymère hydrophile est une albumine sérique humaine pouvant être réticulée ou une protéine sérique humaine pouvant être réticulée, et **en ce que** l'albumine sérique humaine ou la protéine sérique humaine sont fonctionnalisées par des groupes qui sont choisis parmi les groupes maléimide, vinylsulfonique, acrylate, halogénure d'alkyle, azirine, pyridile, acide thionitrobenzoïque ou des groupes arylants.

2. Composition selon la revendication 1, **caractérisée en ce qu'**elle présente en outre au moins un des éléments suivants, à savoir des cellules de mammifères, un agent pharmacologique, un agent biologiquement actif, ou un ou plusieurs ou des mélanges de ceux-ci.

3. Composition selon la revendication 2, **caractérisée en ce que** les cellules de mammifères sont choisies parmi des cellules musculo-squelettiques, en particulier parmi les chondrocytes, les ostéocytes, les fibrochondrocytes, ainsi que parmi des cellules glandulaires impliquées dans la régulation du métabolisme, des cellules d'îlots de Langerhans, des cellules produisant de la mélatonine, des cellules précurseurs et des cellules souches, en particulier des cellules souches mésenchymateuses.

4. Composition selon la revendication 2, **caractérisée en ce que** l'agent pharmacologiquement actif est choisi parmi au moins l'un des éléments suivants : un antibiotique, un anti-inflammatoire, une hormone métabolique, des chondro-protecteurs, des agents servant à la thérapie génique, des hormones de croissance, des facteurs de différenciation ou de modulation, des immunosuppresseurs, des substances immunostimulantes, des DMOA, des acides nucléiques, des principes actifs induisant l'apoptose, des principes actifs améliorant l'adhérence, des agonistes récepteurs et des antagonistes récepteurs ou des mélanges de ceux-ci.

5. Composition selon l'une quelconque des revendications 1 à 4, **caractérisée en ce que** la concentration en albumine dans le gel est comprise entre environ 5 et environ 15 mg/ml de gel, est en particulier d'environ 10 mg/ml de gel.

6. Composition selon l'une quelconque des revendications 1 à 5, **caractérisée en ce que** l'agent biologiquement actif est un acide hyaluronique.

7. Composition selon l'une quelconque des revendications 1 à 6, **caractérisée en ce que** la concentration en acide hyaluronique dans le gel est comprise entre environ 1 et environ 10 mg/ml de gel, est en particulier d'environ 4 mg/ml de gel.

8. Utilisation d'albumine sérique humaine fonctionnalisée ou de protéine sérique humaine fonctionnalisée pour la production d'une composition biocompatible pouvant être injectée, l'albumine sérique humaine ou la protéine sérique humaine étant fonctionnalisées par des groupes qui sont choisis parmi les groupes maléimide, vinylsulfonique, acrylate, halogénure d'alkyle, azirine, pyridile, acide thionitrobenzoïque ou des groupes arylants.

9. Utilisation selon la revendication 8, **caractérisée en ce que** de l'albumine sérique fonctionnalisée maléimide ou de la protéine sérique fonctionnalisée maléimide est utilisée.

10. Utilisation d'une composition selon l'une quelconque des revendications 1 à 7 pour la production de matériaux biodégradables et biocompatibles destinés à être utilisés dans la pharmacologie, la médecine vétérinaire et/ou humaine.

11. Utilisation selon la revendication 10, **caractérisée en ce que** lors de l'utilisation, la composition est utilisée comme tout ou comme composant dans des dispositifs pouvant être implantés ou dans des implants destinés à être utilisés dans le cadre du rétablissement, de la reconstruction et/ou du remplacement de tissus et/ou d'organes, ou en tant qu'implant libérant un médicament pour les mammifères.

12. Utilisation selon la revendication 11, **caractérisée en ce que** lors de l'utilisation, la composition est utilisée pour remplacer et/ou pour régénérer des cartilages articulaires, des disques intervertébraux, des tissus osseux, des cellules servant à la construction de tissus, et des cellules sécrétrices impliquées dans la régulation du métabolisme.

13. Procédé de production d'une composition biocompatible pouvant être injectée comprenant au moins un polymère hydrophile, qui peut être polymérisé in situ en un gel, lequel procédé présente les étapes suivantes consistant à :
a) préparer du sérum humain ou de l'albumine sérique humaine ;
b) fonctionnaliser avec du maléimide, de la sulfone de vinyle, de l'acrylate, de l'halogénure d'alkyle, de l'azirine, du pyridile, de l'acide thionitrobenzoïque, ou des dérivés de ceux-ci, ou des groupes arylants ;
c) éventuellement ajouter des cellules de mammifères, un agent pharmacologique, un agent biologiquement actif ou des mélanges de ceux-ci ; et
d) ajouter un réticulant servant à réticuler la protéine sérique humaine ou l'albumine sérique humaine fonctionnalisées à l'étape b).
